# DEMANDE DE BREVET EUROPEEN

(11) **EP 0 646 383 A1**
(43) Date de publication de la demande: **05.04.1995**
(21) Numéro de dépôt: 94402185.6
(22) Date de dépôt: 29.09.1994
(51) Int. Cl.: A61M 5/36

(54) **Procédé et dispositif de détection de bulles dans une ligne de perfusion**

(30) Priorité: 30.09.1993 FR 9311656
(71) Demandeur: Becton Dickinson and Company, Franklin Lakes, New Jersey 07417-1880 (US)
(72) Inventeur: Saugues, Alain, F-38430 Moirans (FR); Brose, Marc, F-38590 Brezins (FR)
(74) Mandataire: Leszczynski, André

(57) **Abrégé**

Dispositif de détection de bulles dans une ligne de perfusion comportant un premier tube (14) pour transporter un fluide, un piège à bulles (18) comportant un orifice d'admission relié au premier tube, un réceptacle (26) possédant une chambre pouvant être rempli de fluide, et un orifice de sortie et de telle sorte que lorsqu'une bulle pénètre dans la chambre par l'intermédiaire de l'orifice d'admission, un volume équivalent de fluide est déplacé depuis la chambre et quitte le réceptacle par l'intermédiaire de l'orifice de sortie, un second tube (16) relié à l'orifice de sortie du réceptacle, un détecteur de bulles (22) apte à détecter une bulle de longueur prédéterminée dans le second tube, et des moyens pour générer un signal si une telle bulle est détectée.

## Description

La présente invention a trait d'une manière générale au domaine de la détection de bulles dans des canalisations de fluide. Elle a trait plus particulièrement à un détecteur de bulles pour un système de perfusion médicale.

Des fluides sont souvent perfusés dans le corps de malades durant le cours d'un traitement médical au moyen de pompes volumétriques et de pompes à seringue. Ces pompes sont reliées au malade au moyen d'une gaine flexible. Durant la perfusion, il est possible que des bulles d'air se forment dans la gaine. La formation de bulles d'air est indésirable et peut causer des dommages au malade. Les bulles peuvent être de dimensions importantes ou extrêmement petites (appelées "micro-bulles").

Dans l'art antérieur, des bulles ont été détectées au moyen de dispositifs ultrasoniques, optiques et à compression. Ces dispositifs sont utilisés pour déclencher des alarmes ou pour mettre fin à une perfusion si des bulles sont détectées. La détection de micro-bulles par ce procédé est difficile et coûteuse. Si le dispositif doit détecter des bulles de faibles dimensions, un grand nombre de fausses alarmes peut se produire. Si le dispositif détecte uniquement des bulles de grandes dimensions, des fausses alarmes peuvent être réduites, mais au risque de perfusion de micro-bulles. Une seule micro-bulle entraîne peu de risques de dommages. Cependant, il est connu qu'un grand nombre de micro-bulles perfusées dans le corps d'un malade pendant une période de temps, entraîne des dommages.

Une pompe volumétrique de perfusion doit être apte à détecter une bulle ayant un volume d'environ 0,05 ml ou moins. En utilisant des détecteurs de l'art antérieur, une alarme est déclenchée uniquement après une détermination que le volume de bulles dépasse un volume critique. Le temps pour atteindre ce volume critique dépend du débit. Ce procédé contribue ainsi à la génération de fausses alarmes et se traduit par une détection lente de bulles.

Des bulles d'air peuvent être emprisonnées dans une chambre disposée dans la ligne de perfusion. Cependant, un tel piège doit être dimensionné pour assurer qu'il sera apte à recevoir les bulles se formant sur une période de temps prolongée. En résultat, le volume de la ligne de perfusion est accru, se traduisant par une durée accrue d'amorçage et une perte du médicament.

La présente invention concerne un dispositif de détection de bulles dans une ligne de perfusion, par exemple, dans une pompe volumétrique. Un premier tube est prévu pour transporter un fluide. Un piège à bulles est relié au premier tube. Le piège à bulles possède une admission reliée au premier tube, une chambre pouvant être remplie par le fluide, et un orifice de sortie. Lorsqu'une bulle pénètre dans la chambre par l'intermédiaire de l'orifice d'admission, un volume équivalent de fluide est déplacé depuis la chambre et quitte le réceptacle par l'intermédiaire de l'orifice de sortie. Un second tube est relié à l'orifice de sortie du réceptacle. Le second tube traverse un détecteur de bulles apte à détecter une bulle de longueur prédéterminée dans le second tube. Des moyens sont prévus pour générer un signal si une bulle ayant la longueur prédéterminée est détectée par le détecteur de bulles.

La présente invention a également pour objet un procédé de détection de bulles dans un fluide dans un tube. Le procédé comporte les étapes consistant à prévoir un premier tube et un second tube ; à prévoir un piège à bulles possédant un orifice d'admission relié au premier tube et un orifice de sortie relié au second tube ; amorcer le réceptacle avec le fluide ; prévoir un détecteur de bulles de telle sorte que le détecteur de bulles peut détecter un gaz dans le second tube ; provoquer l'écoulement du fluide dans le piège à bulles depuis le premier tube et hors du piège à bulles à travers l'orifice de sortie de telle sorte qu'une bulle dans le fluide dans le premier tube déplace un volume équivalent du fluide dans le piège à bulles lors de son entrée dans le réceptacle ; permettre au piège à bulles d'être sensiblement vidé de fluide de telle sorte que le piège à bulles soit sensiblement rempli du gaz et de telle sorte qu'une partie du gaz quitte le piège à bulles par l'intermédiaire de l'orifice de sortie et pénètre sous forme de bulles dans le second tube ; détecter la quantité de gaz dans le second tube et générer un signal lorsqu'une bulle ayant une longueur prédéterminée est détectée dans le second tube.

Dans le but de mieux faire comprendre l'invention, on va maintenant en décrire un exemple de réalisation non limitatif en se référant aux dessins annexés, sur lesquels :
la Figure 1 est une vue en perspective d'une pompe volumétrique incorporant la présente invention ;
la Figure 2 est une vue en élévation détaillée de la gaine et du système de détection de l'invention ;
les Figures 3a, b et c sont des vues en élévation détaillées représentant le fonctionnement de l'invention;
la Figure 4 est une vue détaillée du piège à bulles de l'invention ;
la Figure 5 est une vue de dessus du piège à bulles de l'invention.

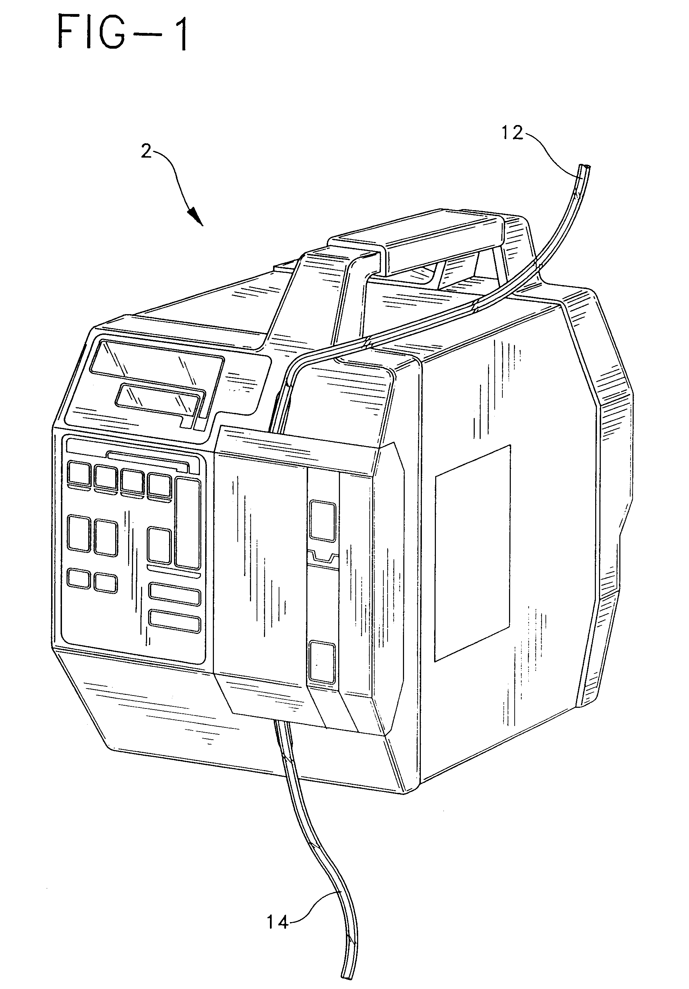

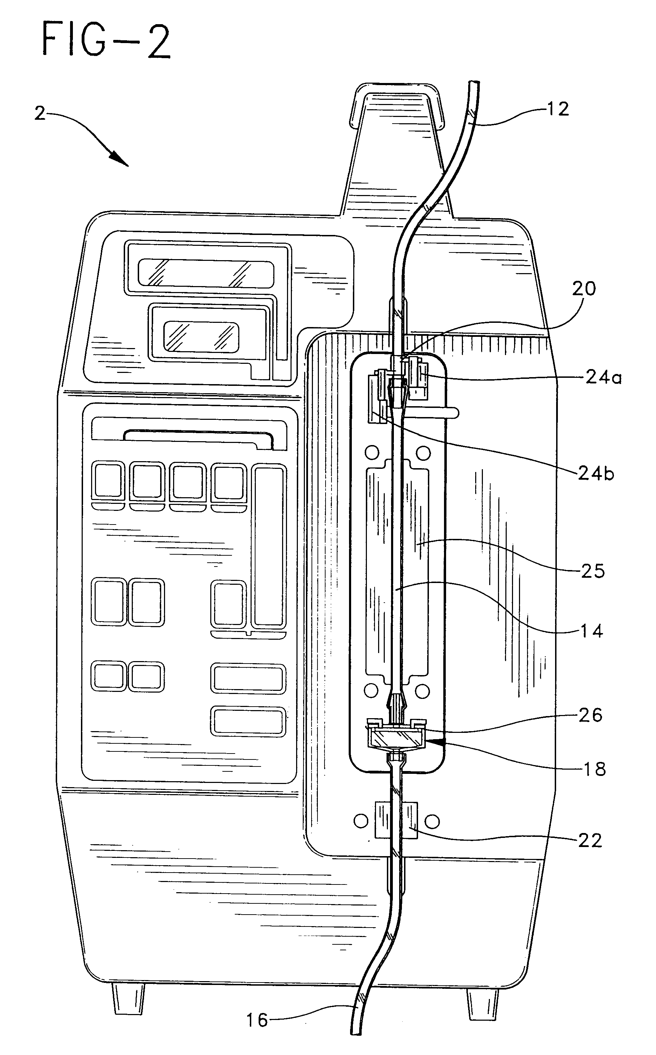

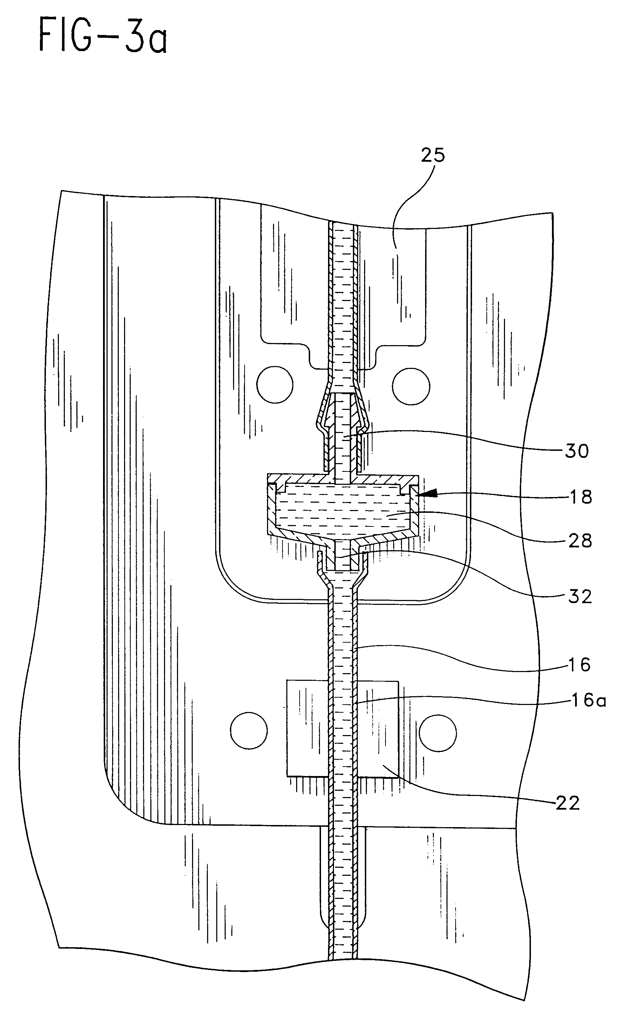

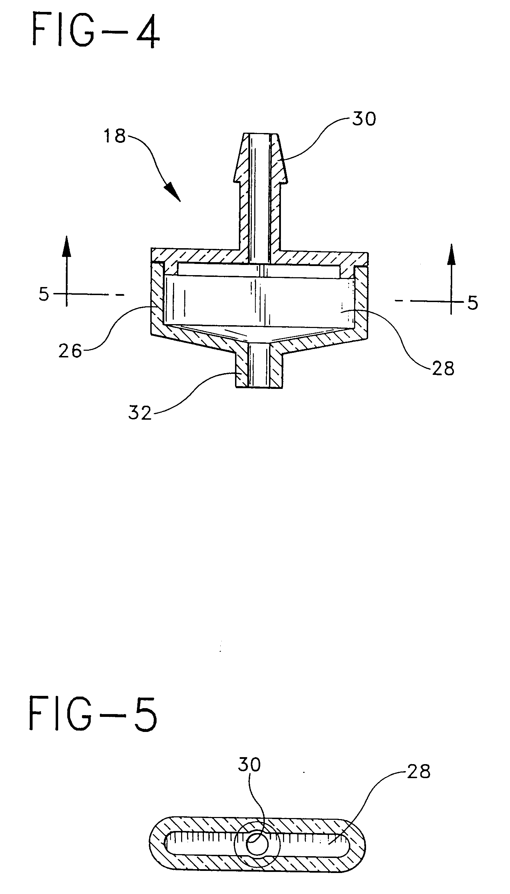

Sur la Figure 1 est représentée une pompe volumétrique 2 incorporant la présente invention. Une telle pompe est disponible auprès de Becton Dickinson Infusion Systems de Brézins, France sous le nom "VIP II". L'invention est représentée plus en détail sur la Figure 2. Le détecteur de bulles 10 est constitué du piège à bulles 18 en série avec le détecteur 22. Le tube 12 est relié à un réservoir de fluide qui doit être perfusé dans le corps d'un malade (non représenté). Le tube 12 est une partie d'un ensemble de perfusion commercialement disponible modifié conçu pour recevoir la pompe VIP Il et disponible auprès de Becton Dickinson Infusion Systems. Un élément de positionnement 20 est relié au tube 12 et agit pour disposer le tube 12 avec précision dans la pompe 2. La pompe 2 est munie de languettes 24a et 24b qui maintiennent le tube 12 en position sur la pompe 2 de sorte qu'elle ne sera pas installée l'arrière à l'avant ou le haut vers le bas. Le tube 12 est typiquement réalisé en PVC. Fixé au tube 12 se trouve un tube 14 qui est typiquement réalisé en caoutchouc aux silicones. La souplesse du caoutchouc aux silicones fait qu'il est bien approprié pour une utilisation dans des pompes volumétriques qui utilisent l'action d'une came ou d'un dispositif péristaltique appuyant sur le tube 14 pour pomper du fluide depuis le réservoir. La pompe VIP II représentée sur les Figures 1 à 5 possède un tel dispositif 25.

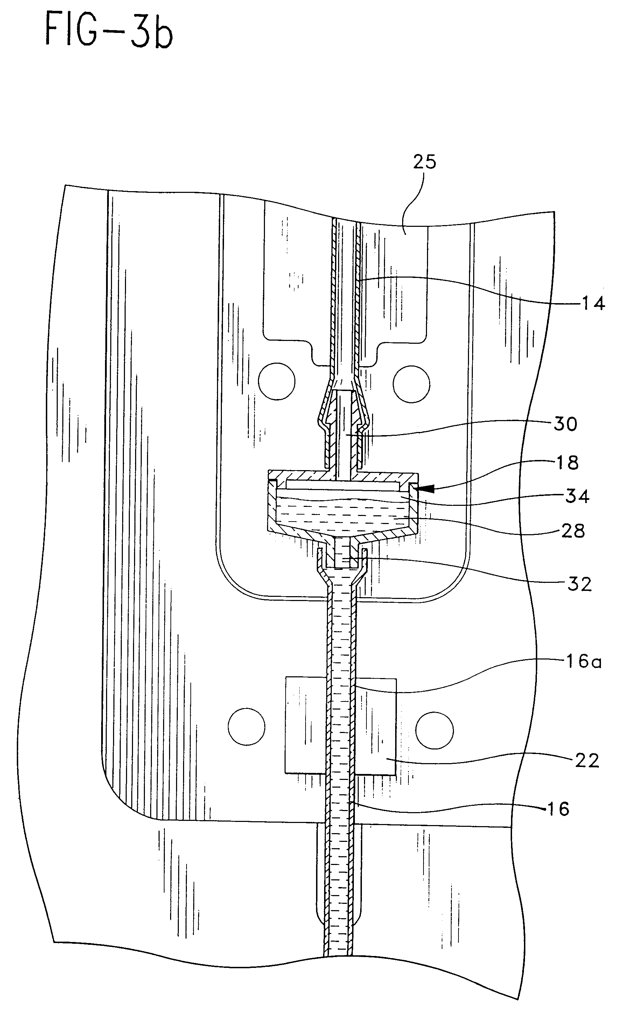

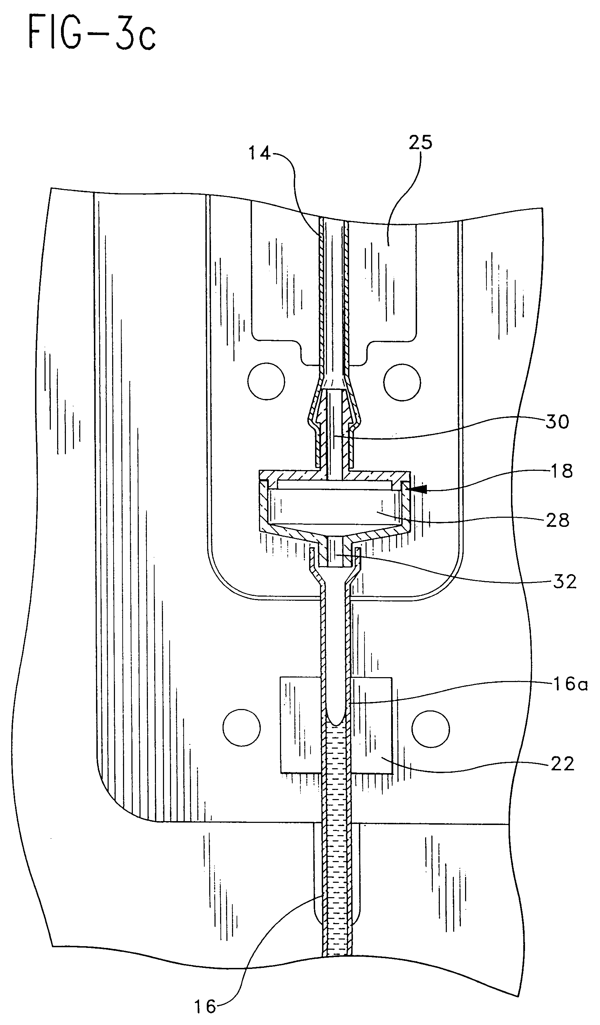

Le tube 14 est fixé à un piège à bulles 18 qui est constitué d'un réceptacle 26 possédant une chambre 28. Le fluide peut s'écouler depuis le tube 14 dans la chambre 28 par l'intermédiaire d'un orifice d'admission 30 qui est relié au tube 14 (cf. Figure 3). Le fluide peut s'échapper de la chambre 28 par l'intermédiaire d'un orifice de sortie 32 qui est relié à un tube 16. Le tube 16 est à son tour relié à un cathéter ou dispositif similaire qui peut être introduit dans un vaisseau sanguin d'un malade pour une perfusion de fluide.

Dans le mode de réalisation préféré, le volume de la chambre 28 est compris approximativement entre environ 0,2 et 0,3 ml. Le tube 16 possède un diamètre intérieur d'environ 3 mm.

Le tube 16 traverse un détecteur de bulles 22 qui est un détecteur de bulles bon marché bien connu dans la technique. Un détecteur de bulles ultrasonique est de préférence utilisé. Un détecteur couramment disponible quelconque peut être utilisé, pourvu qu'il soit apte à détecter une bulle possédant une longueur prédéterminée correspondant à un volume d'environ 0,1 ml pour la configuration préférée décrite ci-dessus. Une telle bulle occupera environ 15 mm du tube. Le détecteur 22 génère un signal qui interrompt le fonctionnement de la pompe 2 lorsqu'une bulle ayant la longueur prédéterminée est détectée. Le détecteur est de préférence étalonné pour détecter des bulles d'un volume sensiblement plus élevé et donc d'une plus grande longueur que sans le piège à bulles.

Lorsque la pompe 2 est placée pour être mise en utilisation, la canalisation de fluide constituée des tubes 12, 14 et 16 est amorcée avec le fluide à perfuser. La chambre 28 est également amorcée avec le fluide. Lorsque la pompe 2 pompe le fluide, le fluide traverse les tubes. Aussi longtemps que du fluide est pompé, la chambre 28 demeure remplie de fluide. Si de l'air ou un autre gaz 34 sous la forme d'une bulle est présent dans le fluide, il pénètre dans la chambre 28 (Cf. Figure 3b). Puisque l'air ou un gaz est moins dense que le fluide, il ne sera pas pompé depuis la chambre 28 dans le tube 16. Au contraire, l'air ou le gaz déplacera un volume équivalent de fluide et sera emprisonné dans la chambre 28. Lorsque plus de bulles pénètrent dans la chambre 28, le fluide dans la chambre 28 sera déplacé (Cf.Figure 3c). Eventuellement, si de nombreuses bulles se déplacent dans la chambre 28, pratiquement la totalité du fluide sera déplacée et uniquement l'air ou le gaz provenant des bulles subsistera. Un autre pompage provoquera au moins un déplacement de l'air ou du gaz vers le bas du tube 16 et dans la région 16a qui est alignée sur le détecteur de bulles 22. Lorsque ceci se produit, le détecteur de bulles 22 génèrera un signal qui peut être utilisé pour arrêter la pompe 2 avant que la bulle pénètre dans le corps du malade.

Le piège à bulles 18 emprisonnera des bulles d'une dimension quelconque. En résultat, même des micro-bulles seront empêchées de pénétrer dans le tube 16 et par suite dans le corps du malade. Le détecteur de bulles 22 peut être un détecteur de bulles à coût relativement faible apte à détecter des bulles de dimensions relativement grandes. Il suffit qu'il soit déclenché lorsque des bulles de grandes dimensions sont présentes du fait qu'il n'est appelé à fonctionner que lorsque la chambre 28 est vide de fluide et qu'une bulle de grandes dimensions est poussée dans le tube 16. Grâce à la présente invention, en particulier à des faibles débits du fait de l'absence de micro-bulles, l'alarme peut être déclenchée immédiatement lorsqu'une bulle est détectée sans le calcul du volume critique nécessaire avec des dispositifs de l'art antérieur. Le volume relativement faible du piège permet cependant à la pompe de générer des alarmes pour des faibles volumes d'air dans la canalisation.

## Revendications

1. Dispositif de détection de bulles dans une ligne de perfusion, caractérisé en ce qu'il comporte:
un premier tube (14) pour transporter un fluide,
un piège à bulles (18) comportant un orifice d'admission (30) relié au premier tube, un réceptacle (26) possédant une chambre (28) pouvant être remplie de fluide, et un orifice de sortie (32), et agencé de telle sorte que lorsqu'une bulle pénètre dans la chambre par l'intermédiaire de l'orifice d'admission, un volume équivalent de fluide est déplacé de la chambre et quitte le réceptacle par l'intermédiaire de l'orifice de sortie,
un second tube (16) relié à l'orifice de sortie du réceptacle,
un détecteur de bulles (22) apte à détecter une bulle de longueur prédéterminée dans le second tube (16), et
des moyens pour générer un signal si une bulle ayant la longueur prédéterminée est détectée.

2. Dispositif selon la revendication 1, caractérisé en ce que le détecteur de bulles (22) se trouve en aval du réceptacle (26) de telle sorte que du fluide peut s'écouler depuis le réceptacle (26) dans le détecteur de bulles.

3. Dispositif selon la revendication 1, caractérisé en ce que le réceptacle (26) est maintenu à une élévation supérieure à celle du détecteur de bulles (22).

4. Dispositif selon la revendication 1, caractérisé en ce que le détecteur de bulles (22) est étalonné pour détecter des bulles d'un volume sensiblement plus grand que sans le piège à bulles.

5. Procédé de détection de bulles dans un fluide dans un tube, les bulles étant constituées d'un gaz, le procédé étant caractérisé en ce qu'il comporte les étapes consistant à:
prévoir un premier tube (14) et un second tube (16),
prévoir un piège à bulles (18) possédant un orifice d'admission (30) relié au premier tube (14), un orifice de sortie (32) relié au second tube (16) et un réceptacle (26),
amorcer le réceptacle (26) avec le fluide,
prévoir un détecteur de bulles (22) apte à détecter un gaz sous forme de bulles dans le second tube,
provoquer l'écoulement du fluide dans le piège à bulles depuis le premier tube et hors du piège à bulles par l'intermédiaire de l'orifice de sortie de telle sorte qu'une bulle dans le fluide dans le premier tube déplace un volume équivalent du fluide dans le piège à bulles lors de son entrée dans le réceptacle,
permettre au piège à bulles d'être pratiquement vide de fluide de sorte que le piège à bulles est sensiblement rempli de gaz et de telle sorte qu'une partie du gaz quitte le piège à bulles par l'intermédiaire de l'orifice de sortie et pénètre sous forme de bulle dans le second tube, et
détecter la partie du gaz sous forme de bulle dans le second tube et générer un signal lorsqu'une bulle ayant une longueur prédéterminée est détectée dans le second tube.

6. Procédé selon la revendication 5, caractérisé en ce que l'étape de détection est effectuée par un détecteur à bulles apte à détecter une bulle d'un volume sensiblement plus élevé que sans piège à bulles.
